# EUROPEAN PATENT APPLICATION

(11) **EP 3 391 867 A1**
(43) Date of publication of application: **24.10.2018**
(21) Application number: 16875721.9
(22) Date of filing: 15.12.2016
(51) Int. Cl.: A61G 15/02

(54) **MEDICAL CARE SYSTEM, MEDICAL CARE DEVICE, COMMUNICATION DEVICE, AND MEDICAL CARE DEVICE OPERATIONAL ERROR PREVENTION METHOD**

(30) Priority: 17.12.2015 JP 2015246204
(71) Applicant: J. Morita Manufacturing Corporation, Kyoto-shi, Kyoto 612-8533 (JP)
(72) Inventor: SONOBE, Kouichi, Kyoto-shi Kyoto 612-8533 (JP); UEJIMA, Yoshio, Kyoto-shi Kyoto 612-8533 (JP); TSURUTA, Yuki, Kyoto-shi Kyoto 612-8533 (JP)
(74) Representative: Müller Hoffmann & Partner
(86) International application number: PCT/JP2016/087368
(87) International publication number: WO 2017/104744

(57) **Abstract**

When a medical care apparatus (10) and a communication device (100) are communicating with each other, a user can make a detailed setting for each function of the medical care apparatus (10) by using the communication device (100). On the other hand, as to the first function by which a smooth medical care action may be directly disturbed depending on setting contents, a setting by the communication device (100) for the first function is restricted, so that it becomes possible to prevent a defect from occurring in the first function by an erroneous operation of the communication device (100).

## Description

### TECHNICAL FIELD

The present invention relates to a medical care system, a medical care apparatus, a communication device, and a method of preventing an erroneous operation of the medical care apparatus.

### BACKGROUND ART

Conventionally, a medical care apparatus for conducting medical care in the healthcare field such as a dental field and a medical field includes an operation unit through which each of a plurality of functions can be set.

For example, Japanese Patent Laying-Open No. 2010-273916 (PTD 1) discloses a dental care unit including an operation panel provided with: a switch for changing the position of a medical care chair; a switch for changing the posture of the medical care chair; and a switch for setting other functions.

### CITATION LIST

### PATENT DOCUMENT

PTD 1: Japanese Patent Laying-Open No. 2010-273916

### SUMMARY OF INVENTION

### TECHNICAL PROBLEM

In the dental care unit disclosed in PTD 1, a user can set various functions by operating a non-liquid crystal-type operation panel that does not include a liquid crystal display. Such an operation panel is inferior in ability to make a detailed setting for each function of the dental care unit, as compared with a touch panel provided on a liquid crystal display. This is because the touch panel provided on the liquid crystal display allows the user to make a detailed setting by combining various operations while switching displays on the screen. However, a liquid crystal display and a touch panel are relatively expensive. Accordingly, when an operation panel of the dental care unit is replaced with a liquid crystal display and a touch panel, the cost of the dental care unit is increased. Also, all of the users do not necessarily desire to use a dental care unit that allows the users to make a detailed setting for each function thereof.

Thus, it is conceivable to use a communication device such as a tablet-type computer provided separately from a dental care unit so as to allow the user to make a detailed setting for each function of the dental care unit. However, in the case where a communication device provided separately from the dental care unit is used to allow the user to make a detailed setting for each function provided in the dental care unit, if the communication device is erroneously operated, a smooth medical care action may be directly disturbed depending on functions.

The present invention has been made to solve the above-described problems. An object of the present invention is to provide a medical care system, a medical care apparatus, a communication device, and a method of preventing an erroneous operation of the medical care apparatus, by which a detailed setting for each function of the medical care apparatus can be made while minimizing influences upon the cost of the medical care apparatus, and by which a direct disturbance to a smooth medical care action by an erroneous operation of the communication device is prevented.

### SOLUTION TO PROBLEM

A medical care system according to the present invention includes: a medical care apparatus having a plurality of functions; and a communication device capable of communicating with the medical care apparatus. The medical care apparatus includes a first operation unit through which each of the plurality of functions can be set. The communication device includes a second operation unit through which at least one of the plurality of functions can be set in a more detailed manner than through the first operation unit. The plurality of functions include specific functions. Settings by the second operation unit for the specific functions are restricted.

Preferably, the medical care apparatus includes a movable unit configured to change at least one of a position and a posture of the medical care apparatus. The specific functions are for driving the movable unit.

Preferably, when the medical care apparatus and the communication device are not communicating with each other, a setting for each of the plurality of functions is allowed in the first operation unit, and a setting for each of the plurality of functions is restricted in the second operation unit. When the medical care apparatus and the communication device are communicating with each other, in the first operation unit, settings for the specific functions among the plurality of functions are allowed and settings for other functions different from the specific functions among the plurality of functions are restricted, and in the second operation unit, the settings for the other functions are allowed and the settings for the specific functions are restricted.

Preferably, the medical care apparatus includes a first visibility changing unit configured to change visibility of the first operation unit. The communication device includes a second visibility changing unit configured to change visibility of the second operation unit. When the medical care apparatus and the communication device are communicating with each other, the first visibility changing unit is configured to lower the visibility of the first operation unit associated with other functions different from the specific functions among the plurality of functions below the visibility of the first operation unit associated with the specific functions, and the second visibility changing unit is configured to lower the visibility of the second operation unit associated with the specific functions below the visibility of the second operation unit associated with the other functions.

Preferably, the communication device is a mobile terminal capable of wirelessly communicating with the medical care apparatus, and configured to communicate with a medical care apparatus among a plurality of medical care apparatuses that is closest to the communication device.

Preferably, the communication device includes a storage unit configured to store setting data about other functions different from the specific functions among the plurality of functions. When the medical care apparatus and the communication device are communicating with each other, the other functions are set in the medical care apparatus based on the setting data stored in the storage unit.

Preferably, the communication device includes an voice authentication unit configured to authenticate a user by voice authentication. The storage unit is configured to store the setting data and information, which allows identification of the user, such that the setting data and the information are associated with each other. When the medical care apparatus and the communication device are communicating with each other, the other functions are set in the medical care apparatus based on the setting data associated with a user authenticated by the voice authentication unit.

Preferably, the communication device includes a facial configuration authentication unit configured to authenticate a user by facial configuration authentication. The storage unit is configured to store the setting data and information, which allows identification of the user, such that the setting data and the information are associated with each other. When the medical care apparatus and the communication device are communicating with each other, the other functions are set in the medical care apparatus based on the setting data associated with a user authenticated by the facial configuration authentication unit.

Preferably, the medical care apparatus includes an attachment unit to which the communication device can be attached. When the communication device is attached to the attachment unit, the medical care apparatus and the communication device are brought into communication with each other.

Preferably, the communication device is configured to update at least one of a program and data of the medical care apparatus when the medical care apparatus and the communication device are communicating with each other.

The medical care apparatus according to the present invention is a medical care apparatus included in the medical care system in any one of the above description.

The communication device according to the present invention is a communication device included in the medical care system in any one of the above description.

A method of preventing an erroneous operation of a medical care apparatus according to the present invention includes: performing an operation by which a setting for each of a plurality of functions can be made; communicating with a communication device by which a setting for at least one of the plurality of functions can be made in a more detailed manner than in the performing an operation; and restricting settings by the communication device for specific functions among the plurality of functions.

### ADVANTAGEOUS EFFECTS OF INVENTION

According to the medical care system of the present invention, a detailed setting for each function of the medical care apparatus is allowed by using a communication device, whereas a setting by the communication device for each specific function of the medical care apparatus is restricted. Accordingly, a detailed setting for each function of the medical care apparatus can be made while minimizing influences upon the cost of the medical care apparatus. Also, it becomes possible to prevent a direct disturbance to a smooth medical care action caused by an erroneous operation of the communication device.

### BRIEF DESCRIPTION OF DRAWINGS

Fig. 1 is a schematic diagram showing a medical care system.
Fig. 2 is a block diagram schematically showing an inner configuration of a medical care apparatus.
Fig. 3 is a block diagram schematically showing an inner configuration of a communication device.
Fig. 4 is a diagram for illustrating icons on an operation panel included in the medical care apparatus.
Fig. 5 is a diagram for illustrating illuminated states of the icons on the operation panel and a screen display of the communication device in the state where the medical care apparatus and the communication device are not communicating with each other.
Fig. 6 is a diagram for illustrating the illuminated states of the icons on the operation panel and the screen display of the communication device in the state where the medical care apparatus and the communication device are communicating with each other.
Fig. 7 is a diagram for illustrating a management table stored in the communication device.
Fig. 8 is a flowchart for illustrating a communication process performed by the communication device.
Fig. 9 is a flowchart for illustrating a data extraction process performed by the communication device.
Fig. 10 is a flowchart for illustrating a setting process performed by the communication device.
Fig. 11 is a flowchart for illustrating a setting process performed by the medical care apparatus.
Fig. 12 is a flowchart for illustrating a setting process during operation that is performed by the medical care apparatus in a modification.

### DESCRIPTION OF EMBODIMENTS

Embodiments according to the present invention will be hereinafter described with reference to the accompanying drawings. In the present embodiment, a medical care apparatus that can be used for a dental care will be explained as one illustrative form of a medical care apparatus. In addition, the medical care apparatus according to the present embodiment is applicable not only to a dental care but also to any type of medical cares and treatments in the fields of ophthalmology, otolaryngology, radiology, and veterinary science, and the like. Also, the medical care includes a diagnosis and a treatment.

### [Configuration of Medical Care System]

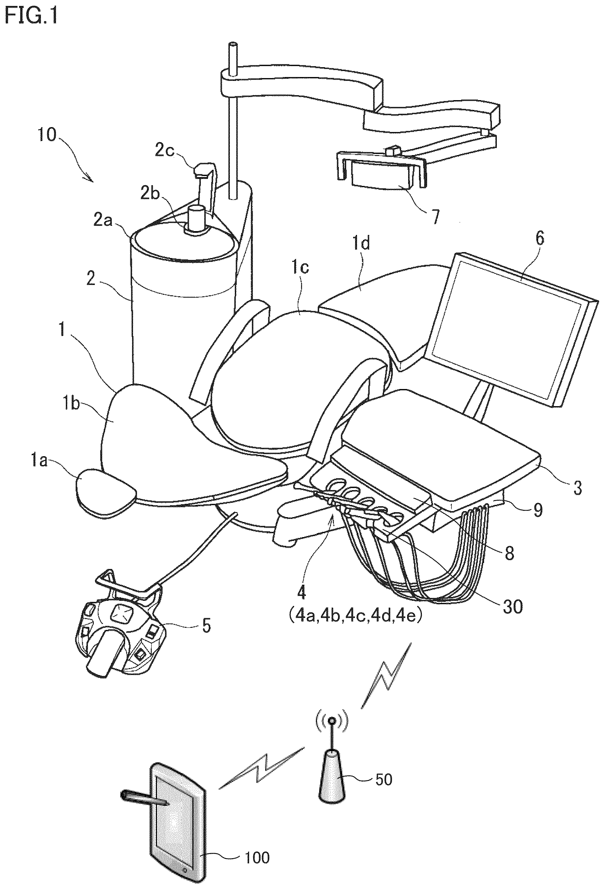

Fig. 1 is a schematic diagram showing a medical care system 1000. As shown in Fig. 1, medical care system 1000 includes a medical care apparatus 10 (which will be hereinafter also referred to as a medical care apparatus 10) and a communication device 100. Communication device 100 is capable of communicating with medical care apparatus 10 in a wireless network environment constructed by an access point 50. In addition, communication device 100 may be able to wirelessly communicate with medical care apparatus 10 without through access point 50. Furthermore, communication device 100 is attached to an attachment unit 45 included in medical care apparatus 10 so as to be wire-connected to medical care apparatus 10. In this case, communication device 100 can communicate with medical care apparatus 10 through a communication line. Although not shown, communication device 100 can wirelessly communicate with a plurality of medical care apparatuses 10. Medical care system 1000 corresponds to one embodiment of the "medical care system"; medical care apparatus 10 corresponds to one embodiment of the "medical care apparatus"; and communication device 100 corresponds to one embodiment of the "communication device" and the "mobile terminal".

### [Configuration of Medical Care Apparatus]

As shown in Fig. 1, medical care apparatus 10 includes a medical care chair 1, a foot controller 5, a tray table 3, a control device 9, an operation panel 8, a basin unit 2, a monitor 6, an attachment unit 45, a lighting device 7, and an instrument holder 30.

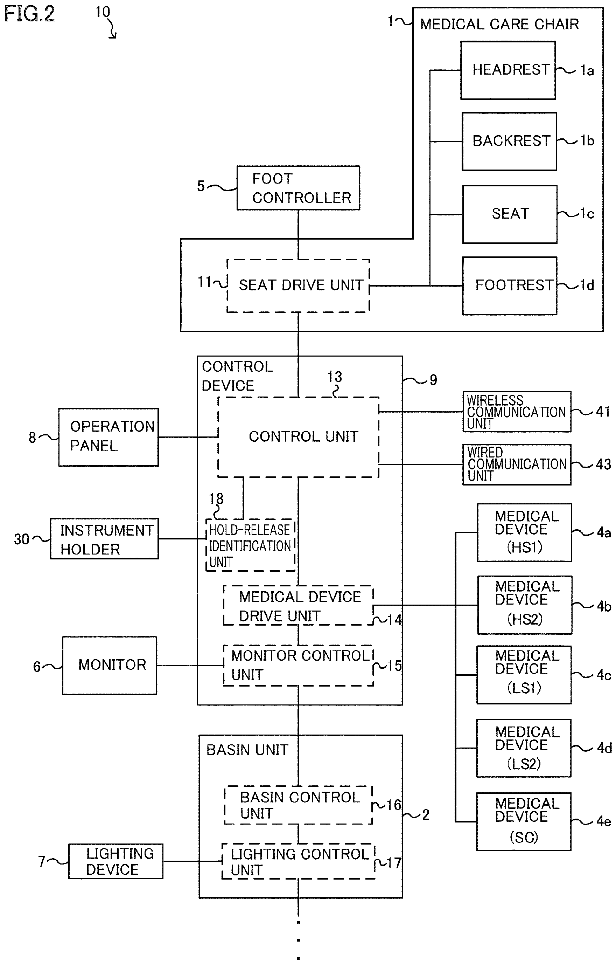

Fig. 2 is a block diagram schematically showing the inner configuration of medical care apparatus 10. As shown in Fig. 2, medical care apparatus 10 includes, as main function units having prescribed functions, a seat drive unit 11, a control unit 13, a medical device drive unit 14, a monitor control unit 15, a basin control unit 16, a lighting control unit 17, and a hold-release identification unit 18. Each of these function units is configured by a central processing unit (CPU), a read only memory (ROM), a random access memory (RAM) (not shown) and the like that are mounted on a substrate.

Medical care chair 1 includes a headrest 1a supporting the head of a patient, a backrest 1b supporting the back of the patient, a seat 1c supporting buttocks of the patient, and a footrest 1d supporting legs of the patient. Each of headrest 1a, backrest 1b, seat 1c, and footrest 1d is driven under the control of seat drive unit 11.

For example, seat 1c is raised or lowered under the control of seat drive unit 11. Each of headrest 1a, backrest 1b and footrest 1d is moved under the control of seat drive unit 11 in the direction that is perpendicular or horizontal to seat 1c. When headrest 1a, backrest 1b and footrest 1d are moved in the direction perpendicular to seat 1c (which is also referred to as standing of medical care chair 1), the patient sitting on medical care chair 1 is brought into a seated posture. When headrest 1a, backrest 1b and footrest 1d are moved in the direction horizontal to seat 1c (which is also referred to as inclination of medical care chair 1), the patient sitting on medical care chair 1 is laid on his/her back.

In this way, seat drive unit 11 has a function of driving each of headrest 1a, backrest 1b, seat 1c, and footrest 1d to change the position and the posture of medical care chair 1. It is to be noted that headrest 1a, backrest 1b, seat 1c, and footrest 1d correspond to one embodiment of the "movable unit".

Foot controller 5 receives the operation for driving medical care chair 1. Also, foot controller 5 receives the operation for driving a medical device 4. The user can operate foot controller 5 by his/her foot stepping.

Upon reception of the operation for driving medical care chair 1, foot controller 5 outputs a control signal to seat drive unit 11. Seat drive unit 11 drives medical care chair 1 based on the control signal. Upon reception of the operation for driving medical device 4, foot controller 5 outputs a control signal to medical device drive unit 14. Medical device drive unit 14 drives medical device 4 based on the control signal.

Tray table 3 is used as a table on which tools and the like are placed during the medical care. Tray table 3 is connected to an arm (not shown) extending from medical care chair 1 or a floor. Accordingly, the user can manually move tray table 3 pivotally, horizontally and perpendicularly relative to medical care chair 1.

Control device 9 is provided on the bottom surface of tray table 3. To this control device 9, a plurality of medical devices 4, operation panel 8, instrument holder 30, monitor 6, a wireless communication unit 41, and a wired communication unit 43 are connected.

Medical device 4 is, for example, an instrument for medical care such as an air turbine handpiece (high-speed handpiece), a micro motor handpiece (low-speed handpiece), a scaler, a three-way syringe, and a vacuum syringe. Medical device 4 is not limited to the above components, but may be an intraoral camera, a photopolymerization irradiator, a root canal length measuring instrument, a root canal enlargement instrument, and the like, or may be non-driving instrument such as a mirror and a syringe.

In medical care apparatus 10, five types of medical devices 4a, 4b, 4c, 4d, and 4e are used. For example, medical devices 4a and 4b each are a high-speed handpiece (also referred to as HS (HS1 and HS2)). Medical devices 4c and 4d each are a low-speed handpiece (referred to as LS (LS1 and LS2)). Medical device 4e is a scaler (also referred to as SC). Each medical device 4 is held by instrument holder 30. When medical device 4 is removed from instrument holder 30, the held state by instrument holder 30 is released, which leads to a state where medical device 4 is selected (also referred to as a selected state).

Each medical device 4 is connected to medical device drive unit 14 included in control device 9. Medical device drive unit 14 drives each medical device 4 based on the user's operation of foot controller 5. For example, HS1 and HS2 each serve to rotate a cutting tool held at a head portion under the control of medical device drive unit 14.

In this way, medical device drive unit 14 has a function of driving medical device 4 based on the user's operation of foot controller 5.

On operation panel 8, a plurality of icons for setting various functions are illuminated. By performing an operation of pressing the illuminated icon on operation panel 8, the user can perform an operation of pressing an inner switch corresponding to this illuminated icon. In addition, operation panel 8 corresponds to one embodiment of the "first operation unit".

Operation panel 8 is connected to control unit 13 included in control device 9. Control unit 13 allows only an icon, which corresponds to a function that can be set, to be illuminated on operation panel 8. Then, when the illuminated icon is pressed, control unit 13 outputs a control signal for executing a function to a function unit connected to a switch corresponding to this illuminated icon.

In this way, control unit 13 causes an icon to be illuminated or unilluminated on operation panel 8 depending on whether a function corresponding to the icon can be set or not, and also causes the function unit, which is connected to the switch corresponding to the pressed icon, to execute its function. In addition, control unit 13 corresponds to one embodiment of the "first visibility changing unit".

Instrument holder 30 serves as a holding member for holding each medical device 4. Instrument holder 30 is connected to hold-release identification unit 18 provided in control device 9. Hold-release identification unit 18 detects a medical device 4 that is held by instrument holder 30 and a medical device 4 that is not held but in the selected state. Then, hold-release identification unit 18 notifies control unit 13 about the detection results.

Monitor 6 is a liquid crystal display, for example. Monitor 6 is connected to monitor control unit 15 provided in control device 9. For example, monitor control unit 15 causes monitor 6 to display: an image about driving of medical device 4 in the selected state; an image showing the position at the tip end of a cutting tool inside a root canal; an image inside the oral cavity taken by an intraoral camera; and the like.

In this way, monitor control unit 15 has a function of causing monitor 6 to display a prescribed image.

Wireless communication unit 41 serves as a wireless antenna through which a radio wave is transmitted/received when medical care apparatus 10 wirelessly communicates with communication device 100. The signal from communication device 100 is conveyed to wireless communication unit 41 via a radio wave transmitted from communication device 100, and then, received by control unit 13. On the other hand, the signal from control unit 13 is conveyed to communication device 100 via a radio wave transmitted from wireless communication unit 41.

Wired communication unit 43 serves as an interface that establishes wired connection between medical care apparatus 10 and communication device 100 when communication device 100 is attached to attachment unit 45 (see Fig. 1). The signal from communication device 100 is transmitted from communication device 100 through a communication line, and received by control unit 13. On the other hand, the signal from control unit 13 is transmitted from wired communication unit 43 through a communication line to communication device 100. In addition, attachment unit 45 corresponds to one embodiment of the "attachment unit".

As shown in Fig. 1, basin unit 2 is provided on the side portion of medical care chair 1. Basin unit 2 includes a bowl 2a provided with an exhaust port, a cup stand 2b on which a cup is placed, and a water faucet 2c for supplying water to a cup. The patient can gargle with water supplied into the cup from water faucet 2c. As shown in Fig. 2, basin unit 2 includes a basin control unit 16. Basin control unit 16 is configured to control the flow of water that is to be used in medical care apparatus 10.

In this way, basin control unit 16 has a function of controlling the flow of the water used in medical care apparatus 10.

Furthermore, basin unit 2 includes a lighting control unit 17. Lighting control unit 17 is configured to control lighting device 7 to be turned on and off.

In this way, lighting control unit 17 has a function of controlling lighting device 7 to be turned on and off (also referred to as a lighting control function).

In addition, medical care apparatus 10 may include a configuration other than the configuration shown in Fig. 1, and a function unit other than the function units shown in Fig. 2.

### [Configuration of Communication Device]

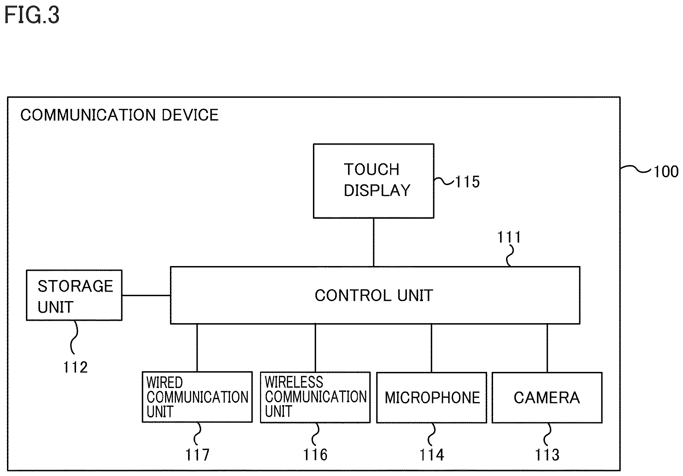

Communication device 100 in the present embodiment is a tablet-type mobile terminal that the user can carry. As shown in Fig. 3, communication device 100 includes a control unit 111, a storage unit 112, a camera 113, a microphone 114, a touch display 115, a wireless communication unit 116, and a wired communication unit 117.

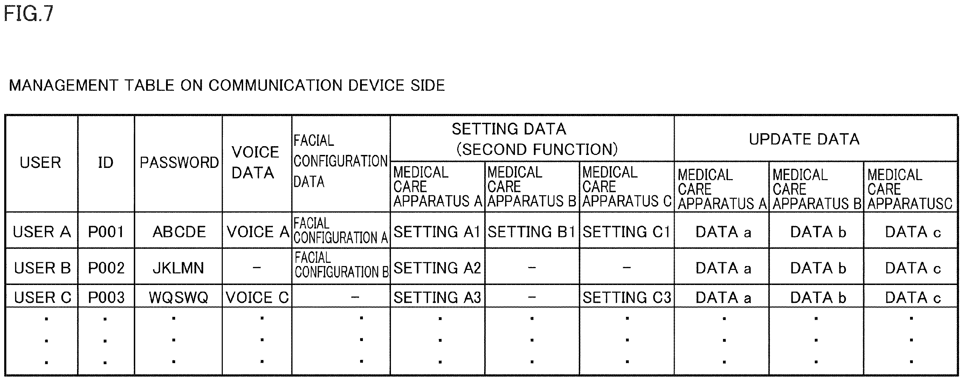

Control unit 111 is configured by a CPU and the like mounted on the substrate. Storage unit 112 is formed of a ROM or a RAM, and configured to store a management table in which the information and the like about the user are stored. Control unit 111 executes various processes while referring to the management table stored in storage unit 112. The management table will be described later with reference to Fig. 7. In addition, storage unit 112 corresponds to one embodiment of the "storage unit".

Touch display 115 has a liquid crystal display provided with a touch panel. On the liquid crystal display, various switches are displayed as icon images. The user can perform an operation of pressing the icon image displayed on touch display 115. Each icon image is switched to be displayed and not-displayed under the control of control unit 111. When an icon image is pressed, control unit 111 executes the process corresponding to the operation indicated by the icon image. Touch display 115 corresponds to one embodiment of the "second operation unit". Also, control unit 111 corresponds to one embodiment of the "second visibility changing unit".

Camera 113 takes an image of an object and outputs the image data of the obtained image to control unit 111. Control unit 111 executes a prescribed process based on the image data from camera 113. For example, control unit 111 performs a facial configuration authentication process for determining whether the image data corresponding to a user's facial configuration image taken by camera 113 and the facial configuration data stored in advance in storage unit 112 match with each other or not. Control unit 111 corresponds to one embodiment of the "facial configuration authentication unit".

Microphone 114 converts the voice input from the outside into an electrical signal, and outputs the signal to control unit 111. Control unit 111 recognizes the voice based on the electrical signal from microphone 114, and performs a prescribed process based on the recognized voice. For example, control unit 111 performs a voice authentication process for determining whether the voice data corresponding to the user's voice collected by microphone 114 and the voice data stored in advance in storage unit 112 match with each other or not. Control unit 111 corresponds to one embodiment of the "voice authentication unit".

Wireless communication unit 116 serves as a wireless antenna through which a radio wave is transmitted/received during wireless communication with medical care apparatus 10. The signal from medical care apparatus 10 is conveyed to wireless communication unit 116 via a radio wave transmitted from medical care apparatus 10, and then received by control unit 111. On the other hand, the signal from control unit 111 is conveyed to medical care apparatus 10 via a radio wave transmitted from wireless communication unit 116.

Wired communication unit 43 serves as an interface that establishes wired connection between communication device 100 and medical care apparatus 10 when communication device 100 is attached to attachment unit 45 of medical care apparatus 10. The signal from medical care apparatus 10 is transmitted from medical care apparatus 10 through a communication line and then received by control unit 111. On the other hand, the signal from control unit 111 is transmitted from wired communication unit 43 through a communication line to medical care apparatus 10.

In addition, the state of communicating with each other means a state where a signal can be transmitted/received between medical care apparatus 10 and communication device 100 via wired communication or wireless communication. Also, the state of not communicating with each other means a state where a signal cannot be transmitted/received between medical care apparatus 10 and communication device 100 via wired communication or wireless communication.

### [Configuration of Operation Panel]

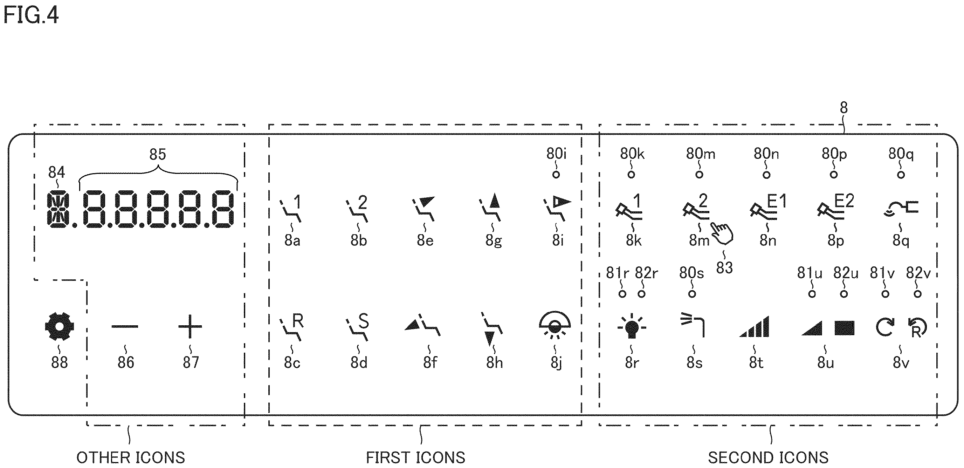

The configuration of operation panel 8 will be hereinafter described with reference to Fig. 4. Fig. 4 is a diagram for illustrating icons on operation panel 8 included in medical care apparatus 10.

Inside operation panel 8, switches (not shown) for enabling the functions of the function units are arranged on the substrate. A plate-shaped panel is provided so as to cover the upper surface of the substrate. The panel has a surface on which a plurality of icons are silk-printed with ink made of a light diffusing material. When these icons are illuminated from an LED (not shown) provided inside operation panel 8, the illuminated icons appear on operation panel 8. Thereby, the icons become visible to the user. When the user presses an icon on operation panel 8, the user can perform an operation to press a switch, which is provided inside the panel so as to correspond to the pressed icon. In addition, appearance of an icon on operation panel 8 is also represented as a state where an icon is illuminated, and disappearance of an icon from operation panel 8 is also represented as a state where an icon is unilluminated.

Each switch provided inside operation panel 8 is a switch that can be physically pressed, but not a virtual switch such as an icon image displayed on a screen. Accordingly, the user can obtain a feeling of pressing a switch (a click feeling) when the user presses the illuminated icon that is silk-printed on operation panel 8.

Inside operation panel 8, switches corresponding to all function units, respectively, are provided. However, the icons corresponding to all of the switches are not necessarily illuminated on operation panel 8 so as to be always visible, but only the icon corresponding to the function that can be set (can be executed) at that time is illuminated.

For example, when an icon corresponding to a raise switch is illuminated on operation panel 8 even though medical care chair 1 cannot be raised anymore, the user may misunderstand that medical care chair 1 can still be raised. Thus, when medical care chair 1 becomes unable to be raised anymore, the icon corresponding to the raise switch is to be unilluminated on operation panel 8. Thereby, it becomes possible to prevent the user from erroneously operating the switch corresponding to the function unit for the function that cannot be set anymore.

Specifically, control unit 13 of medical care apparatus 10 controls an LED disposed around each of switches provided in operation panel 8 to thereby stop illumination of the icon corresponding to the switch for a function that cannot be set anymore. Thereby, when the function cannot be set anymore, the visibility of the icon is lowered to the level at which the icon cannot be recognized by the user, below the level at the time when the function can still be set.

In this way, the switch for a function that cannot be set even by operating this switch is eliminated from the user's sight or is made difficult to be seen from the user, thereby preventing the user from operating a switch for a function that cannot be set anymore.

Then, the icons displayed on operation panel 8 will be hereinafter described with reference to Fig. 4. Fig. 4 is a plan view of operation panel 8 and shows the state where all of the icons are illuminated on operation panel 8.

An icon 8a, an icon 8b, an icon 8c, and an icon 8d each are pressed for setting a function of moving medical care chair 1 to a prescribed position by one operation (also referred to as a chair auto function). The chair auto function is one of the functions executed by seat drive unit 11.

The switches for executing the chair auto function includes: an auto 1 switch for automatically moving, by one operation, medical care chair 1 to a position at which medical care chair 1 is set at a height and at an inclination angle that are suitable for care for back teeth; an auto 2 switch for automatically moving, by one operation, medical care chair 1 to a position at which medical care chair 1 is set at a height and at an inclination angle that are suitable for care for front teeth; an auto R switch for automatically moving, by one operation, medical care chair 1 to a position at which medical care chair 1 is set at a height and at an inclination angle that are suitable for entry and exit of the patient; and an auto S switch for automatically moving, by one operation, medical care chair 1 to a position at which medical care chair 1 is set at a height and at an inclination angle that are suitable for the patient to perform gargling. Icon 8a corresponds to the auto 1 switch. Icon 8b corresponds to the auto 2 switch. Icon 8c corresponds to the auto R switch. Icon 8d corresponds to the auto S switch.

In addition, the user can arbitrarily set the position to which medical care chair 1 is automatically moved by operating the auto 1 switch, the auto 2 switch, or the auto S switch.

An icon 8e, an icon 8f, an icon 8g, and an icon 8h each are pressed when setting a function of continuously changing one of the position and the posture of medical care chair 1 (which is also referred to as a chair manual function). The chair manual function is one of the functions of seat drive unit 11.

The switches for executing the chair manual function include: a stand-up switch for continuously raising medical care chair 1 to stand according to the operation time (the time during which the user presses an icon); an inclination switch for continuously inclining medical care chair 1 according to the operation time; a raise switch for continuously raising medical care chair 1 according to the operation time; and a lower switch for continuously lowering medical care chair 1 according to the operation time. Icon 8e corresponds to the stand-up switch. Icon 8f corresponds to the inclination switch. Icon 8g corresponds to the raise switch. Icon 8h corresponds to the lower switch.

In addition, the chair manual function may be a function of gradually moving at least one of the position and the posture of medical care chair 1. In this case, the switch for activating the chair manual function is to gradually move at least one of the position and the posture of medical care chair 1 according to the number of times of operations (the number of times of pressing an icon). In this way, the switch for executing the chair manual function allows the chair manual function to be continuously or gradually executed according to the duration time of pressing an icon or the number of times of pressing an icon.

Icon 8i is pressed when setting a function of setting the movement speed of medical care chair 1 (which is also referred to as a chair speed function). Icon 8i corresponds to a chair speed switch. When the user presses the chair speed switch for a relatively long time, the movement speed of medical care chair 1 is switched between the normal speed and the low speed.

A dot icon 80i is placed above icon 8i. Dot icon 80i shows the setting state of the movement speed of medical care chair 1. For example, when the movement speed of medical care chair 1 is set at a normal speed, dot icon 80i is unilluminated. When the movement speed of medical care chair 1 is set at a low speed, dot icon 80i is illuminated.

Icon 8j is pressed when setting the function of switching lighting device 7 to be turned on and off (which is also referred to as a lighting function). Icon 8j corresponds to a lighting switch. Lighting device 7 is changed to be turned on and off each time the user operates the lighting switch.

Each of Icons 8k and 8m, which shows the attachment state of a high-speed handpiece (HS), is pressed when selecting the attached HS (HS1 and HS2). Icon 8k corresponds to an HS1 switch. Icon 8m corresponds to an HS2 switch. When the user operates the HS1 switch or when the user removes the HS1 from instrument holder 30 and releases holding of HS1, the HS1 is brought into a selected state. When the user operates the HS2 switch or when the user removes the HS2 from instrument holder 30 and releases holding of the HS2, the HS2 is brought into a selected state.

A dot icon 80k and a dot icon 80m are arranged above icon 8k and icon 8m, respectively. Dot icon 80k shows the selected state and the driven state of the HS1. Dot icon 80m shows the selected state and the driven state of the HS2. For example, when the HS1 is in a selected state, dot icon 80k is illuminated. While the HS1 is driven, dot icon 80k blinks.

Each of Icons 8n and 8p, which shows the attachment state of a low-speed handpiece (LS), is pressed when selecting the attached LS (LS1 and LS2). Icon 8n corresponds to an LS1 switch. Icon 8p corresponds to an LS2 switch. When the user operates the LS1 switch or when the user removes the LS1 from instrument holder 30 and releases holding of the LS1, the LS1 is brought into a selected state. When the user operates the LS2 switch or when the user removes the LS2 from instrument holder 30 and releases holding of the LS2, the LS2 is brought into a selected state.

A dot icon 80n and a dot icon 80p are arranged above icon 8n and icon 8p, respectively. Dot icon 80n shows the selected state and the driven state of the LS 1. Dot icon 80p shows the selected state and the driven state of the LS2. For example, when the LS1 is in a selected state, dot icon 80n is illuminated. While the LS1 is driven, dot icon 80n blinks.

An icon 8q, which shows the attachment state of a scaler (SC), is pressed when selecting the attached SC. Icon 8q corresponds to an SC switch. When the user operates the SC switch or when the user removes the SC from instrument holder 30 and releases holding of the SC, the SC is brought into a selected state.

A dot icon 80q is arranged above icon 8q. Dot icon 80q shows the selected state and the driven state of the SC. For example, when the SC is in a selected state, dot icon 80q is illuminated. While the SC is driven, dot icon 80q blinks.

An icon 8r is pressed when an HP light (not shown) is switched to be turned ON and OFF. The HP light is provided at the tip end portion of medical device 4 and configured to emit light to the area around the tip end portion. The light emitted from the HP light is, for example, a medium for medical care used for illuminating the affected part. Icon 8r corresponds to an HP light switch. An LED light is sequentially switched among the brightly illuminated state, the darkly illuminated state and the unilluminated state each time the user operates the HP light switch.

Dot icons 81r and 82r are arranged above icon 8r. Each of dot icons 81r and 82r shows the illuminated state of the HP light. For example, when the HP light is brightly illuminated, dot icon 81r and dot icon 82r are illuminated. When the HP light is darkly illuminated, only dot icon 81r is illuminated. When the HP light is turned off, dot icons 81r and 82r are unilluminated.

An icon 8s is pressed when water supply by an HP water supply unit (not shown) is switched to be started and stopped. The HP water supply unit is provided at the tip end portion of medical device 4 and configured to emit water to the area around the tip end portion. The water emitted from the HP water supply unit is, for example, a medium for medical care used for cooling the affected part. The medium for medical care used for cooling the affected part is not limited to water but may be air. Icon 8s corresponds to an HP water supply switch. Water supply by the HP water supply unit is switched to be started and stopped each time the user operates the HP water supply switch.

A dot icon 80s is arranged above icon 8s. Dot icon 80s shows the state where water is supplied by the HP water supply unit. For example, when the HP water supply unit is turned on, dot icon 80s is illuminated. When the HP water supply unit is turned off, dot icon 80s is unilluminated.

An icon 8t is pressed when changing the setting range of the rotation speed of the LS and the setting range of the driving force of the SC. Icon 8t corresponds to a range changing switch. The setting range of the rotation speed of the LS or the setting range of the driving force of the SC is changed each time the user operates the range changing switch.

In addition, an icon 84 shows the setting range changed by the operation of the range changing switch. For example, when the LS is selected, characters of "U" (Under), "L" (Low), "M" (Middle), and "H" (High) are indicated as icon 84 in order of narrowness of the setting range of the rotation speed. When the SC is selected, characters of "P" (Perio), "E" (Endo), "S" (Scaling), and "B" (Boost) are indicated as icon 84 in order of narrowness of the setting range of the driving force. Furthermore, icon 85 indicates the maximum rotation speed of the LS or the maximum driving force of the SC in accordance with the setting range. In addition, when the user operates a negative switch corresponding to icon 86, the rotation speed of the LS and the driving force of the SC can be set to be low. Also, when the user operates a positive switch corresponding to icon 87, the rotation speed of the LS and the driving force of the SC can be set to be high.

An icon 8u is pressed when the rotation speed mode of each of the HS and the LS is switched between a fixed speed mode and an variable speed mode. The fixed speed mode is a mode in which a cutting tool at the tip end of each of the HS and the LS rotates at a determined and fixed speed. The variable speed mode is a mode in which the cutting tool at the tip end of each of the HS and the LS rotates at a speed in accordance with the depressed amount of foot controller 5. Icon 8u corresponds to a fixed speed/variable speed changing switch. The rotation speed mode of each of the HS and the LS is switched between the fixed speed mode and the variable speed mode each time the user operates the fixed speed/variable speed changing switch.

A dot icon 81u and a dot icon 82u are arranged above icon 8u. Dot icon 81u and dot icon 82u each show the state of the rotation speed mode. For example, when the rotation speed mode is a variable speed mode, only dot icon 81u is illuminated. When the rotation speed mode is a fixed speed mode, only dot icon 82u is illuminated.

An icon 8v is pressed when the rotation direction mode of the LS is switched between the normal rotation mode and the reverse rotation mode. The normal rotation mode is a mode in which the cutting tool at the tip end of the LS rotates in the clockwise direction. The reverse rotation mode is a mode in which the cutting tool at the tip end of the LS rotates in the counter-clockwise direction. Icon 8v corresponds to a rotation direction changing switch. Each time the user operates the rotation direction changing switch, the rotation direction mode of the LS is switched between the normal rotation mode and the reverse rotation mode.

A dot icon 81v and a dot icon 82v are arranged above icon 8v. Dot icon 81v and dot icon 82v each show the state of the rotation direction mode. For example, when the rotation direction mode is a normal rotation mode, only dot icon 81v is illuminated. When the rotation direction mode is a reverse rotation mode, only dot icon 82v is illuminated.

An icon 83 shows that the switch has been operated. When a switch provided in advance corresponding to each icon is operated, icon 83 indicates an icon corresponding to this operated switch.

An icon 88 corresponds to another switch for making a setting other than the settings made by the above-described various switches. In the present embodiment, a switch corresponding to icon 88 is not provided.

In this way, in the present embodiment, a non-liquid crystal type operation panel not employing a liquid crystal display is employed, and the reason thereof will be described below. Specifically, in the case of a liquid crystal-type operation panel, the user's operation of pressing a switch is detected when the user only slightly touches an extremely thin film. This prevents the user from effectively getting a click feeling at the time when the user presses the switch, in contrast to the switch physically pressed by the user as in the present embodiment. Among elderly users, there are some who cannot think that he/she has completed the setting if he/she cannot get a click feeling in response to the switch operation. Accordingly, in order to ensure such a click feeling, it is preferable to use an operation panel equipped with a switch that is physically pressed by the user without using a liquid crystal-type operation panel. In addition, a liquid crystal display and a touch panel are relatively expensive. Accordingly, when a liquid crystal-type operation panel is employed, the cost for medical care apparatus 10 is increased. Furthermore, when a liquid crystal-type operation panel is employed, a detailed setting can be made by combining various operations while changing the display of the screen. This requires the user to touch a liquid crystal display many times during the medical care, which is not preferable for maintaining the hygienic conditions.

However, in the case of non-liquid crystal type operation panel 8, settings cannot be readily made as compared with the operation panel having a screen on which a switch is operated, for example, like a liquid crystal type operation panel having a touch panel. Although not all of the users desire to use medical care apparatus 10 that can make a detailed setting for each function, some of the users may desire to perform a meticulous care with the ability to make a detailed setting for each function of the apparatus.

Thus, in the present embodiment, communication device 100 provided separately from medical care apparatus 10 is used to thereby allow a detailed setting for each function of medical care apparatus 10 to be made by remote control. However, if detailed settings for all of the functions of medical care apparatus 10 can be made by communication device 100 provided separately from medical care apparatus 10, when communication device 100 is erroneously operated, a smooth medical care action may be directly disturbed depending on functions.

For example, icons 8a to 8h correspond to switches for setting the functions of seat drive unit 11 for changing at least one of the position and the posture of medical care chair 1. If communication device 100 can remotely operate even such a function of changing the position and the posture of medical care chair 1, when communication device 100 is erroneously operated, medical care chair 1 may be suddenly moved to the position and the posture that the user does not intend, with the result that a smooth medical care action may be directly disturbed. Furthermore, icon 8j corresponds to a switch for setting the lighting function of lighting control unit 17 configured to switch lighting device 7 to be turned on and off If communication device 100 can remotely operate even such the lighting function of lighting device 7, when communication device 100 is erroneously operated, the user's sight suddenly becomes dark during the medical care, with the result that a smooth medical care action may be directly disturbed.

In consideration of the above description, in the present embodiment, a detailed setting for each function of medical care apparatus 10 by using communication device 100 is allowed. On the other hand, as to specific functions among the plurality of functions, detailed settings for these specific functions by communication device 100 are restricted. This will be hereinafter described in detail.

### [Detailed Setting by Communication Device]

In the following description, the first function represents specific functions that may directly disturb a smooth medical care action depending on setting contents; and the second function represents a function that may not directly disturb a smooth medical care action or may be less likely to directly disturb a smooth medical care action. As described above, the first function includes: the chair auto function indicated by icons 8a to 8d; the chair manual function indicated by icons 8e to 8h; the chair speed function indicated by icon 8i, and the lighting function indicated by icon 8j. Also, the second function includes each of functions indicated by icons 8k to 8v. For example, icons 8k to 8q are pressed when selecting medical device 4. However, medical device 4 is not driven only by operating these icons. This medical device 4 in the selected state is not driven until foot controller 5 is operated by foot stepping. Accordingly, even if icons 8k to 8q are erroneously operated, a smooth medical care action may be less likely to be directly disturbed.

It may be considered appropriately as to whether the above-described functions are regarded as the first function or the second function. For example, the lighting function indicated by icon 8j may be regarded less likely to directly disturb a smooth medical care action, and thus, can be classified into the second function. The first function corresponds to one embodiment of the "specific functions" while the second function corresponds to one embodiment of "other functions". Furthermore, icons 8a to 8j are also referred to as the first icons while icons 8k to 8v corresponding to other functions are also referred to as the second icons. In addition, since icons 84 to 87 are icons other than the first icons and the second icons, these icons 84 to 87 are also referred to as other icons.

The user brings communication device 100 close to medical care apparatus 10 to a range in which wireless communication can be established, thereby allowing wireless communication to be established between communication device 100 and medical care apparatus 10. Furthermore, the user attaches communication device 100 to attachment unit 45 of medical care apparatus 10, thereby allowing wired communication to be established between communication device 100 and medical care apparatus 10 through a communication line. When medical care apparatus 10 and communication device 100 are communicating with each other, communication device 100 is operated so as to allow a detailed setting for each function of medical care apparatus 10 to be made.

It should be noted that communication device 100 stores the program and the data for setting the second function, but does not store the program and the data for setting the first function. Accordingly, when communication device 100 and medical care apparatus 10 are communicating with each other, a detailed setting for the second function can be made through remote control by communication device 100, but a detailed setting for the first function cannot be made through remote control by communication device 100.

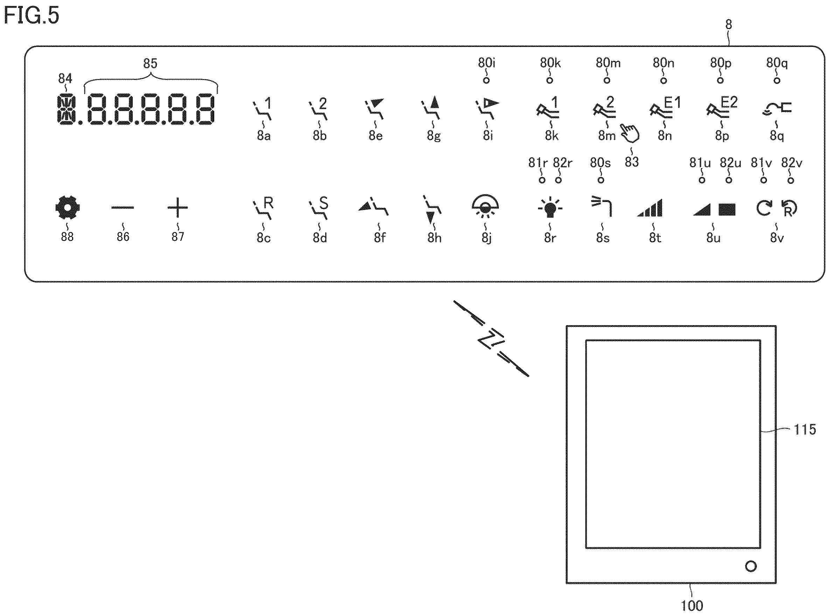

For example, Fig. 5 is a diagram for illustrating the illuminated states of the icons on operation panel 8 and a screen display of communication device 100 in the state where medical care apparatus 10 and communication device 100 are not communicating with each other.

As shown in Fig. 5, when medical care apparatus 10 and communication device 100 are not communicating with each other, the first icons and the second icons are illuminated on operation panel 8 of medical care apparatus 10. On the other hand, setting images for the first function and the second function are not displayed on touch display 115 of communication device 100. In other words, when medical care apparatus 10 and communication device 100 are not communicating with each other, the settings for the plurality of functions are allowed on operation panel 8 of medical care apparatus 10, but the settings for the plurality of functions are restricted on touch display 115 of communication device 100. The term "restrict" herein means "inhibit". In the present embodiment, the setting images are not displayed on touch display 115 of communication device 100, and also, the setting process for each of the plurality of functions is not performed in response to the pressing operation performed on touch display 115.

In addition, on touch display 115 of communication device 100, at least only the setting image of each function of medical care apparatus 10 is not displayed, but other images may be displayed.

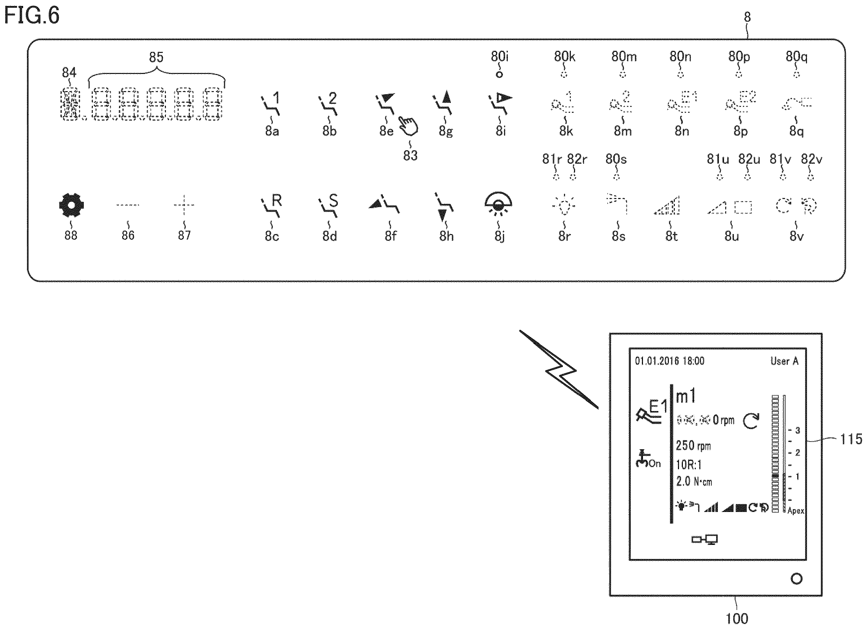

Fig. 6 is a diagram for illustrating the illuminated states of the icons on operation panel 8 and the screen display of communication device 100 in the state where medical care apparatus 10 and communication device 100 are communicating with each other.

As shown in Fig. 6, when medical care apparatus 10 and communication device 100 are communicating with each other, on operation panel 8 of medical care apparatus 10, the first icons are illuminated but the second icons and other icons are unilluminated. On the other hand, on touch display 115 of communication device 100, only the setting image for the second function is displayed but the setting image for the first function is not displayed. In other words, when medical care apparatus 10 and communication device 100 are communicating with each other, among the plurality of functions, the setting for the first function is allowed but the setting for the second function is restricted on operation panel 8 of medical care apparatus 10. The term "restrict" herein means "inhibit". Thus, in the present embodiment, the second icons are not illuminated on operation panel 8 of medical care apparatus 10, and also, the setting process for the second function is not performed in response to the pressing operation performed on operation panel 8. Furthermore, on touch display 115 of communication device 100, the setting for the second function is allowed but the setting for the first function is restricted. The term "restrict" herein means "inhibit". Thus, in the present embodiment, the setting image for the first function is not displayed on touch display 115 of communication device 100, and also, the setting process for the first function is not performed in response to the pressing operation performed on touch display 115.

In this way, a detailed setting for each function of medical care apparatus 10 is allowed by using communication device 100 while the setting by communication device 100 for the first function of medical care apparatus 10 is restricted. Thus, a detailed setting for each function of medical care apparatus 10 can be made while minimizing influences upon the cost of medical care apparatus 10. Also, it becomes possible to prevent a direct disturbance to a smooth medical care action caused by an erroneous operation of communication device 100.

Furthermore, when a detailed setting for the second function is allowed in communication device 100, a detailed setting for the second function is disallowed in medical care apparatus 10, so that the settings in communication device 100 and the settings in medical care apparatus 10 do not overlap with each other. Thereby, it becomes possible to prevent the second function from being set based on the contents that the user does not intend.

### [Automatic Setting by Communication Device]

In the present embodiment, the setting for the second function can be stored in communication device 100 in advance. Thus, when communication device 100 is brought into communication with medical care apparatus 10, the second function can be automatically set in medical care apparatus 10 based on the setting contents stored in advance, which will be hereinafter described in detail.

The user can cause storage unit 112 of communication device 100 to store an ID and a password in advance. Furthermore, the user can communicate with a plurality of medical care apparatuses 10 (in the present embodiment, a medical care apparatus A, a medical care apparatus B, and a medical care apparatus C) using communication device 100. Also, the user can cause storage unit 112 to store, in advance, the setting data about the second function of each medical care apparatus 10. In addition, the user can make a detailed setting separately for each of the plurality of second functions in one of medical care apparatuses 10, and also can cause storage unit 112 to store all of the set data in advance. The setting data of the second function stored in storage unit 112 is associated with an ID and a password.

Furthermore, the user inputs his/her voice into microphone 114 of communication device 100, so as to allow storage unit 112 to store the voice data in advance. The setting of the second function stored in storage unit 112 is associated with the voice data.

Furthermore, the user can cause storage unit 112 to store, in advance, the image data of his/her facial configuration image taken by camera 113. The setting of the second function stored in storage unit 112 is associated with the image data of the facial configuration image.

Furthermore, the user can cause storage unit 112 to store, in advance, the update data for updating at least one of the program and the data in each of the plurality of medical care apparatuses 10. The program corresponds, for example, to an instruction and a processing procedure (software) for each function unit of medical care apparatus 10 to execute a prescribed function. Data corresponds to various pieces of data such as setting data, image data and voice data that are used by each function unit of medical care apparatus 10 to execute a prescribed function. In addition, communication device 100 may obtain update data from a storage medium such as a CD, or may obtain update data from an outer server via wired communication or wireless communication.

For example, Fig. 7 is a diagram for illustrating a management table stored in communication device 100. As shown in Fig. 7, the management table stores IDs, passwords, voice data, facial configuration data, setting data of the second function for each medical care apparatus 10, and update data for each medical care apparatus 10 so as to correspond to each user. When one of the above-mentioned items including the users, the IDs, the passwords, the voice data, the facial configuration data, the setting data of the second function, and the update data is newly recorded in communication device 100, the information stored in this management table is updated.

Thereby, even when a plurality of users share one communication device 100, the update data and the setting data of the second function recorded for each user are extracted from storage unit 112 through authentication by an ID and a password, voice authentication by the voice input to microphone 114, or facial configuration authentication by a facial configuration taken by camera 113. Then, when communication device 100 and medical care apparatus 10 are communicating with each other, the setting data and the update data extracted from storage unit 112 are transmitted to medical care apparatus 10. On the other hand, upon reception of the setting data from communication device 100, medical care apparatus 10 automatically sets the second function based on the received setting data. Furthermore, upon reception of the update data from communication device 100, medical care apparatus 10 updates at least one of the program and the data based on the received update data.

In this way, by setting the second function in advance in storage unit 112 of communication device 100, the second function can be automatically set in medical care apparatus 10 when medical care apparatus 10 and communication device 100 are communicating with each other.

Furthermore, at least one of the program and the data of medical care apparatus 10 can be automatically updated using communication device 100. In addition, during automatic update by communication device 100, communication device 100 may be caused to receive the information about the states of a sensor, an actuator and the like in medical care apparatus 10.

### [Communication Process (Communication Device Side)]

Referring to a flowchart, a medical care system 1000 having the above-described configuration will be hereinafter described with regard to processes performed in communication device 100 and medical care apparatus 10.

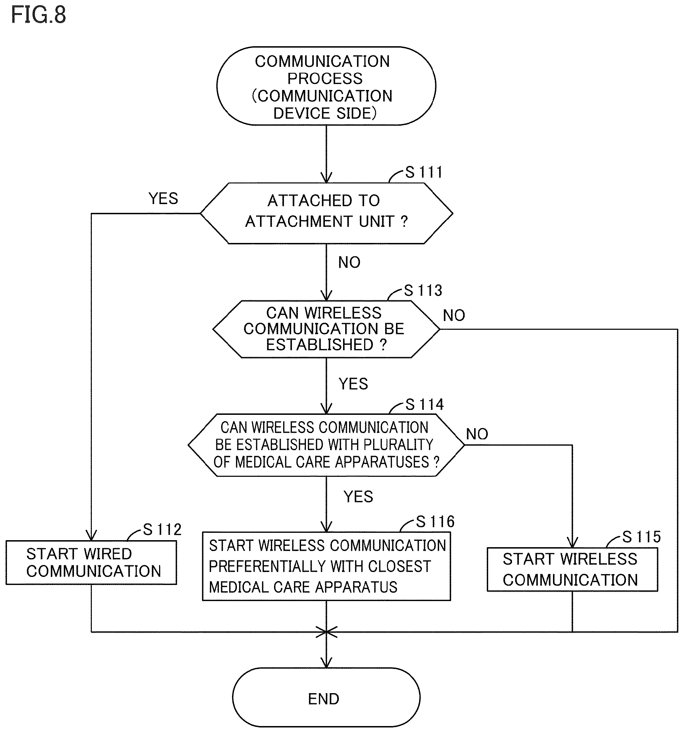

Fig. 8 is a flowchart for illustrating a communication process performed by communication device 100. It is to be noted that the communication process shown in Fig. 8 is a process executed by control unit 111 included in communication device 100 in a prescribed cycle.

As shown in Fig. 8, communication device 100 determines whether it is attached or not to attachment unit 45 of one of the plurality of medical care apparatuses 10 (S111). When communication device 100 is attached to attachment unit 45 of one of medical care apparatuses 10 (YES in S111), communication device 100 starts wired communication with this one medical care apparatus 10 through a communication line (S112), and ends the present routine.

On the other hand, when communication device 100 is not attached to attachment unit 45 of any of medical care apparatuses 10 (NO in S111), this communication device 100 determines whether it can wirelessly communicate with one of medical care apparatuses 10 or not (S113). If communication device 100 cannot wirelessly communicate with any of medical care apparatuses 10 (NO in S113), it ends the present routine.

On the other hand, if communication device 100 can wirelessly communicate with one of medical care apparatuses 10 (YES in S113), communication device 100 determines whether it can wirelessly communicate with the plurality of medical care apparatuses 10 or not (S114). In other words, communication device 100 determines whether the plurality of medical care apparatuses 10 are present or not in a range in which wireless communication can be established.

If communication device 100 cannot wirelessly communicate with the plurality of medical care apparatuses 10, that is, communication device 100 can wirelessly communicate with only one medical care apparatus 10 (NO in S114), communication device 100 starts wireless communication with this one medical care apparatus 10 (S115), and ends the present routine.

On the other hand, if communication device 100 can wirelessly communicate with the plurality of medical care apparatuses (YES in S114), it starts wireless communication preferentially with the closest medical care apparatus 10 (S116), and ends the present routine.

### [Data Extraction Process (Communication Device Side)]

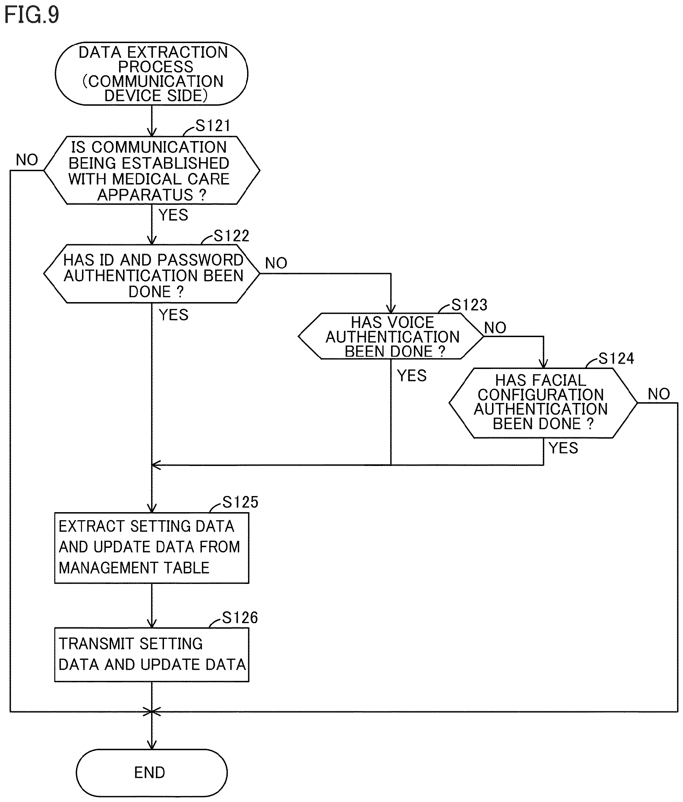

Fig. 9 is a flowchart for illustrating a data extraction process performed by communication device 100. The data extraction process shown in Fig. 9 is a process executed by control unit 111 included in communication device 100 in a prescribed cycle.

As shown in Fig. 9, communication device 100 determines whether it is communicating with medical care apparatus 10 or not via one of wired communication and wireless communication (S121). If communication device 100 is not communicating with medical care apparatus 10 (NO in S121), it ends the present routine.

On the other hand, if communication device 100 is communicating with medical care apparatus 10 (YES in S121), it determines whether an ID and a password have been authenticated or not (S122). Specifically, based on the results of comparing the ID and the password entered by the user with the ID and the password stored in storage unit 112, communication device 100 determines whether the ID and the password have been authenticated or not.

If the ID and the password have not been authenticated (NO in S122), communication device 100 determines whether voice authentication has been done or not (S123). Specifically, based on the results of comparing the user's voice input through microphone 114 with the voice corresponding to the voice data stored in storage unit 112, communication device 100 determines whether the user's voice has been authenticated or not.

If voice authentication has not been done (NO in S123), communication device 100 determines whether facial configuration authentication has been done or not (NO in S124). Specifically, based on the results of comparing the user's facial configuration taken by camera 113 with the facial configuration corresponding to the facial configuration data stored in storage unit 112, communication device 100 determines whether the user's facial configuration has been authenticated or not.

If none of ID and password authentication, voice authentication and facial configuration authentication have been done (NO in S124), communication device 100 ends the present routine.

On the other hand, if one of ID and password authentication (YES in S122), voice authentication (YES in S123) and facial configuration authentication (YES in S124) has been done, communication device 100 extracts, from the management table in storage unit 112, the update data and the setting data of the second function associated with medical care apparatus 10 with which communication device 100 is currently communicating (S125). Then, communication device 100 transmits the extracted setting data and update data to medical care apparatus 10 with which communication device 100 is currently communicating (S126), and then ends the present routine. If the update data and the setting data of the second function associated with currently communicating medical care apparatus 10 are not stored in advance, communication device 100 skips the processes in S125 and S126, and ends the present routine.

### [Setting Process (Communication Device Side)]

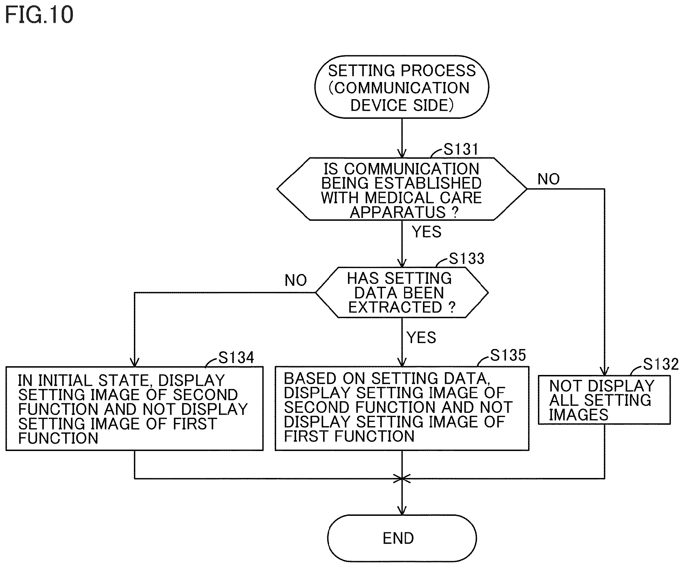

Fig. 10 is a flowchart for illustrating a setting process performed by communication device 100. In addition, the setting process shown in Fig. 10 is a process executed by control unit 111 included in communication device 100 in a prescribed cycle.

As shown in Fig. 10, communication device 100 determines whether it is communicating with medical care apparatus 10 or not via one of wired communication and wireless communication (S131). If communication device 100 is not communicating with medical care apparatus 10 (NO in S131), communication device 100 does not allow display of the setting images of the first function and the second function (S132). In this case, the setting images of the first function and the second function are not displayed on touch display 115 of communication device 100. Accordingly, the user cannot set each function of medical care apparatus 10. In the process in S132, only the setting images of the first function and the second function provided in medical care apparatus 10 are not displayed, but other images may be displayed. Then, communication device 100 ends the present routine.

On the other hand, if communication device 100 is communicating with medical care apparatus 10 (YES in S131), communication device 100 determines whether the setting data has been extracted or not in the data extraction process (S133). If the setting data has not been extracted (NO in S133), in the initial state, communication device 100 allows display of the setting image of the second function and does not allow display of the setting image of the first function (S134). In this case, since only the setting image of the second function is displayed in the initial setting on touch display 115 of communication device 100, the user can set only the second function but cannot set the first function. Then, communication device 100 ends the present routine.

On the other hand, if the setting data has been extracted (YES in S133), based on the setting data, communication device 100 allows display of the setting image of the second function and does not allow display of the setting image of the first function (S135). In this case, only the setting image of the second function is displayed on touch display 115 of communication device 100 based on the setting contents stored by the user in advance. Then, communication device 100 ends the present routine.

### [Setting Process (Medical Care Apparatus Side)]

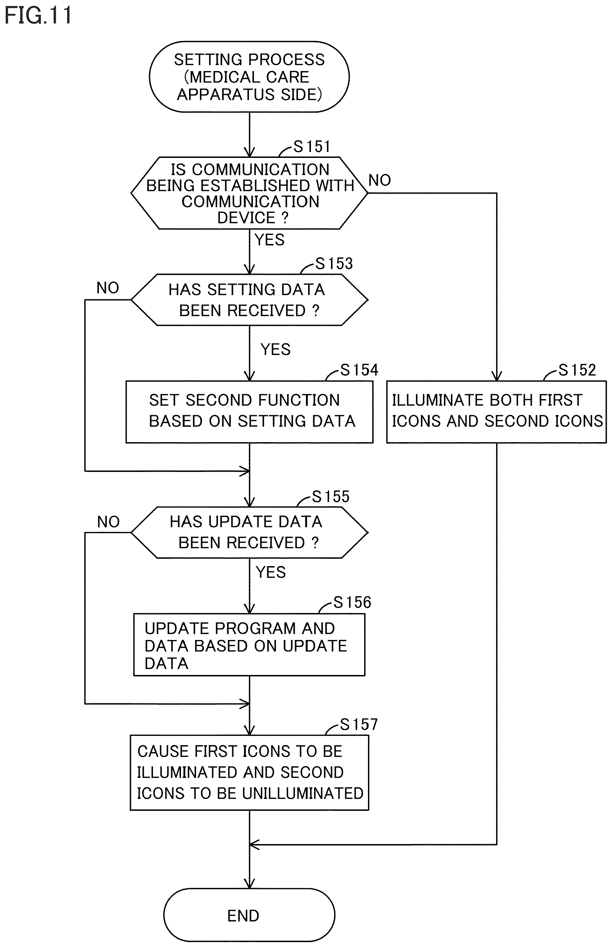

Fig. 11 is a flowchart for illustrating a setting process performed by medical care apparatus 10. The setting process shown in Fig. 11 is a process executed by control unit 13 included in medical care apparatus 10 in a prescribed cycle.

As shown in Fig. 11, medical care apparatus 10 determines whether it is communicating with communication device 100 or not via one of wired communication and wireless communication (S151). If medical care apparatus 10 is not communicating with communication device 100 (NO in S151), it causes the first icons and the second icons to be illuminated on operation panel 8 (S152). In this case, since both the first icons and the second icons are illuminated on operation panel 8, the user can make settings for both the first function and the second function. Then, medical care apparatus 10 ends the present routine.

On the other hand, if medical care apparatus 10 is communicating with communication device 100 (YES in S151), medical care apparatus 10 determines whether it has received the setting data from communication device 100 or not (S153). If medical care apparatus 10 has received the setting data from communication device 100 (YES in S153), it makes a setting for the second function based on the setting data (S154). Thereby, the second function is automatically set based on the contents that are set in communication device 100.

If medical care apparatus 10 has not received the setting data (NO in S153), or after the process in S154 has been done, medical care apparatus 10 determines whether it has received the update data from communication device 100 or not (S155). If medical care apparatus 10 has received the update data from communication device 100 (YES in S155), it updates at least one of the program and the data based on the update data (S156). Thereby, at least one of the program and the data is automatically updated based on the update data stored in communication device 100.

If medical care apparatus 10 has not received the update data (NO in S155), or after the process in S156 has been done, it causes the first icons to be illuminated and the second icons to be unilluminated (S157). In this case, since only the first icons are illuminated on operation panel 8, the user can make a setting only for the first function but cannot make a setting for the second function. Then, medical care apparatus 10 ends the present routine.

As described above, in the present embodiment, communication device 100 is used to allow a detailed setting for each function of medical care apparatus 10 to be made, but a setting by communication device 100 for the specific functions (the first function in the present embodiment) of medical care apparatus 10 is restricted. Accordingly, a detailed setting for each function of medical care apparatus 10 can be made while minimizing influences upon the cost of medical care apparatus 10. Also, it becomes possible to prevent a direct disturbance to a smooth medical care action caused by an erroneous operation of communication device 100.

As to the function of seat drive unit 11 for driving headrest 1a, backrest 1b, seat 1c, and footrest 1d for changing at least one of the position and the posture of medical care apparatus 10, the setting by communication device 100 for this function is restricted. Accordingly, it becomes possible to prevent the position and the posture of medical care apparatus 10 from being changed in an unintentional manner by an erroneous operation of communication device 100.

In the state where medical care apparatus 10 and communication device 100 are not communicating with each other, only the medical care apparatus 10 side has authority to set both the first function and the second function. On the other hand, in the state where medical care apparatus 10 and communication device 100 are communicating with each other, only the medical care apparatus 10 side has authority to set the first function while only the communication device 100 side has authority to set the second function. Accordingly, it becomes possible to prevent a defect from occurring in the first function by an erroneous operation of communication device 100, and also becomes possible to allow a detailed setting for the second function to be made by communication device 100.

In the state where medical care apparatus 10 and communication device 100 are communicating with each other, the first icons are illuminated while the second icons are unilluminated on operation panel 8 of medical care apparatus 10. Then, on touch display 115 of communication device 100, the setting image of the second function is displayed while the setting image of the first function is not displayed. Thereby, it becomes possible to allow the user to visually recognize that, when medical care apparatus 10 and communication device 100 are communicating with each other, a setting for the first function is allowed on the medical care apparatus 10 side but is restricted on the communication device 100 side.

Then, communication device 100 is brought into communication with one medical care apparatus 10 among the plurality of medical care apparatuses 10 that is located closest to this communication device 100. Thus, only by bringing communication device 100 closer to medical care apparatus 10 as a target to be set, this medical care apparatus 10 and communication device 100 can be brought into communication with each other.

In the state where medical care apparatus 10 and communication device 100 are communicating with each other, the second function is set in medical care apparatus 10 based on the setting data stored in storage unit 112 of communication device 100. Thereby, if the second function is set in advance in storage unit 112 of communication device 100, the second function can be automatically set in medical care apparatus 10 when medical care apparatus 10 and communication device 100 are communicating with each other.

In the state where medical care apparatus 10 and communication device 100 are communicating with each other, the second function is set in medical care apparatus 10 based on the setting data associated with the user for which voice authentication has been done. Thereby, the second function can be automatically set in medical care apparatus 10 based on the setting data associated with the user for which voice authentication has been done.

In the state where medical care apparatus 10 and communication device 100 are communicating with each other, the second function is set in medical care apparatus 10 based on the setting data associated with the user for which facial configuration authentication has been done. Thereby, the second function can be automatically set in medical care apparatus 10 based on the setting data associated with the user for which facial configuration authentication has been done.

When communication device 100 is attached to attachment unit 45 of medical care apparatus 10, medical care apparatus 10 and communication device 100 are brought into communication with each other through a communication line. Thereby, it becomes possible to prevent occurrence of inconvenience that medical care apparatus 10 used for medical care is different from medical care apparatus 10 as a target to be set using communication device 100.

In the state where medical care apparatus 10 and communication device 100 are communicating with each other, at least one of the program and the data in medical care apparatus 10 is updated based on the update data stored in storage unit 112 of communication device 100. Accordingly, at least one of the program and the data of medical care apparatus 10 can be automatically updated using communication device 100.

### [Modifications]

The present invention is not limited to the above-described embodiments, but further various modifications and applications thereof can be implemented. The following is an explanation about modifications applicable to the present invention.

### [As to Specific Functions to be Restricted]

In the present embodiment, specific functions for which the settings by communication device 100 are restricted are applied as the first function such as a chair auto function, a chair manual function, a chair speed function, and a lighting function, which may directly disturb a smooth medical care action depending on setting contents. However, the specific functions are not limited to the above, but may be the second function, and the setting by communication device 100 even for the second function may be restricted. Furthermore, any of the functions belonging to the first function may be excluded from the specific functions. For example, only the lighting function of the first function may be regarded less likely to directly disturb a smooth medical care action, and thus, may be excluded from the specific functions.

Furthermore, from among the plurality of functions, the user may be able to select the specific functions for which the settings by communication device 100 are restricted. Furthermore, the user may be able to select, for each medical care apparatus 10, the specific functions for which the settings by communication device 100 are to be restricted. For the purpose of not directly disturbing a smooth medical care action, it is preferable that a setting by communication device 100 at least for the first function is restricted.

### [As to Restriction Method]

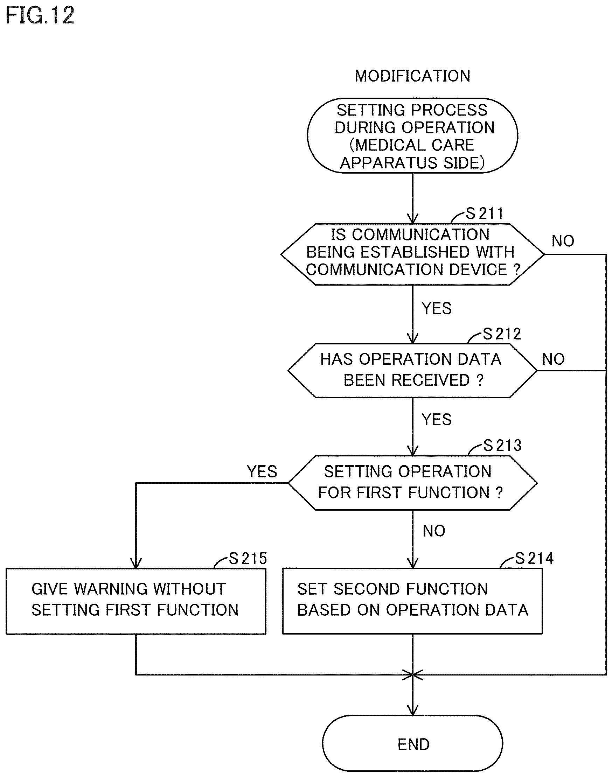

In the present embodiment, the program and the data for setting the first function are not stored in communication device 100. Consequently, no setting image for the first function is displayed on touch display 115, so as to prevent the first function setting operation from being performed through touch display 115. However, without being limited to the above, the setting image for the first function may be displayed on touch display 115 such that the operation itself for setting the first function may be able to be performed through touch display 115. In this case, the setting for the second function only has to be restricted while preventing the operation data associated with the setting operation for the first function from being transmitted to medical care apparatus 10. Furthermore, even if the operation data associated with the setting operation for the first function is to be transmitted to medical care apparatus 10, as in the setting process during operation of medical care apparatus 10 in Fig. 12, the first function may be not set in medical care apparatus 10 based on the operation data associated with the setting operation for the first function.

Fig. 12 is a flowchart for illustrating a setting process during operation that is performed by medical care apparatus 10 in a modification. The setting process during operation shown in Fig. 12 is a process executed by control unit 13 included in medical care apparatus 10 in a prescribed cycle.

As shown in Fig. 12, medical care apparatus 10 determines whether it is communicating with communication device 100 or not via one of wired communication and wireless communication (S211). If medical care apparatus 10 is not communicating with communication device 100 (NO in S211), it ends the present routine.

On the other hand, if medical care apparatus 10 is communicating with communication device 100 (YES in S211), medical care apparatus 10 determines whether it has received the operation data from communication device 100 or not (S212). If medical care apparatus 10 has not received the operation data from communication device 100 (NO in S212), it ends the present routine.

On the other hand, if medical care apparatus 10 has received the operation data from communication device 100 (YES in S212), it determines whether the operation data is the data based on the setting operation for the first function or not (S213). If the setting operation for the first function has not been performed, that is, if the setting operation for the second function has been performed (NO in S213), medical care apparatus 10 sets the second function based on the operation data (S214), and ends the present routine.

On the other hand, if the setting operation for the first function has been performed (YES in S213), medical care apparatus 10 gives a warning sound to the user without setting the first function (S215), and then, ends the present routine. Thereby, even if the operation itself for setting the first function can be performed by communication device 100, the setting for the first function is restricted in medical care apparatus 10. In addition, the process in medical care apparatus 10 for allowing a plurality of functions to be set using operation panel 8 corresponds to one embodiment of the "operation step". Furthermore, the process in medical care apparatus 10 for communicating with communication device 100 corresponds to one embodiment of the "communication step". Furthermore, the process in medical care apparatus 10 for performing step S215 in the setting process during operation in Fig. 12 corresponds to one embodiment of the "restriction step".

In this way, according to the setting process during operation in medical care apparatus 10, a detailed setting for each function of medical care apparatus 10 is allowed by using communication device 100, whereas the setting by communication device 100 for the first function of medical care apparatus 10 is restricted. Accordingly, a detailed setting for each function of medical care apparatus 10 can be made while minimizing influences upon the cost of medical care apparatus 10. Also, it becomes possible to prevent a direct disturbance to a smooth medical care action caused by an erroneous operation of communication device 100.

In the present embodiment, in the state where medical care apparatus 10 and communication device 100 are communicating with each other, the first icons are illuminated while the second icons are unilluminated on operation panel 8 of medical care apparatus 10. However, without being limited to the configuration in which the second icons are completely invisible from the user in this way, the second icons may be lower in visibility than the first icons, for example, in such a manner that the second icons are less visible than the first icons. Furthermore, in the state where medical care apparatus 10 and communication device 100 are communicating with each other, on touch display 115 of communication device 100, the setting image of the second function is displayed while the setting image of the first function is not displayed. However, without being limited to the configuration in which the setting image of the first function is completely invisible from the user in this way, the setting image of the first function may be lower in visibility than the setting image of the second function, for example, in such a manner that the setting image of the first function is less visible than the setting image of the second function.

### [As to Management Table of Communication Device]

In the present embodiment, the setting data of the first function is not stored in the management table of communication device 100, but without being limited thereto, the setting data of the first function may be stored in management table. For example, the setting data of the first function used during medical care (for example, the setting data of the position and the posture of medical care chair 1) may be transmitted from medical care apparatus 10 to communication device 100, and then, the setting data of the first function may be stored in communication device 100. Furthermore, the next time when communication device 100 communicates with medical care apparatus 10, the stored setting data of the first function may be transmitted from communication device 100 to medical care apparatus 10, thereby automatically setting the first function in medical care apparatus 10. However, also in this case, it is preferable that the setting for the first function by operating touch display 115 of communication device 100 is restricted.

In the present embodiment, the update data and the setting data of the second function are stored in storage unit 112 of communication device 100, but the update data and the setting data of the second function may be stored in an outer server that is separately provided. In this case, when communication device 100 communicates with medical care apparatus 10, the update data and the setting data of the second function may be transmitted from the outer server to medical care apparatus 10.

### [As to Automatic Setting by Communication Device]

In the present embodiment, when one of ID and password authentication, voice authentication and facial configuration authentication is performed, the setting data and the update data are transmitted from communication device 100 to medical care apparatus 10, and then, the setting of the second function and the update of the program and the like are to be automatically performed in medical care apparatus 10. However, not only ID and password authentication, voice authentication and facial configuration authentication but also other authentication processes such as fingerprint authentication may be performed, or only one of these authentication processes may be performed. Furthermore, automatic setting may not be performed until all of these authentications are completed.

Also, in the case of automatic setting, the setting data and the update data are transmitted from communication device 100 to medical care apparatus 10, but the setting data and the update data may be stored in advance also in medical care apparatus 10. In this case, when an authentication process is performed, an instruction signal for performing the setting of the second function and the update of the program and the like may be transmitted from communication device 100 to medical care apparatus 10, and then, medical care apparatus 10 may be automatically set based on the setting data and the update data that have been stored according to the instruction signal.

In the present embodiment, the setting of the second function and the update of the program and the like are automatically performed when communication device 100 is brought into communication with medical care apparatus 10. However, when communication device 100 is brought into communication with medical care apparatus 10, the use may select whether the setting of the second function and the update of the program and the like are performed automatically or manually. Alternatively, the user may be able to select the automatic setting or the manual setting in advance.

In the present embodiment, in the case where communication device 100 becomes capable of wirelessly communicating with a plurality of medical care apparatuses 10, communication device 100 starts communication preferentially with the closest medical care apparatus 10. However, the user may be able to select medical care apparatus 10 with which communication device 100 starts communication. Also in this case, when medical care apparatus 10 selected by the user is communicating with another communication device 100, a warning sound may be issued, or the communication with this medical care apparatus 10 may be stopped.

### [As to Communication Device]

In the present embodiment, communication device 100 is a tablet-type mobile terminal but may be other types of communication devices. For example, communication device 100 may be a desktop-type personal computer. Specifically, communication device 100 may be a device that allows a dedicated touch-sensitive panel liquid crystal display to be connected as an accessory to medical care apparatus 10, and also allows a detailed setting for each function of medical care apparatus 10 to be made using this liquid crystal display. In this case, communication device 100 in the present embodiment is replaced with a liquid crystal display as an accessory.

In addition, a transparent cover may be placed on communication device 100 in order to maintain the hygienic conditions during medical care.

### [As to Update of Program or Data]

The present embodiment provides a configuration in which at least one of the program and the data of medical care apparatus 10 is updated through communication between medical care apparatus 10 and communication device 100, but may be a configuration in which update of the program is inhibited while update of the data is allowed.

It should be construed that the embodiments disclosed herein are illustrative and non-restrictive in every respect. The scope of the present invention is defined by the terms of the claims, rather than the description above, and is intended to include any modifications within the meaning and scope equivalent to the terms of the claims.

### REFERENCE SIGNS LIST

1 medical care chair, 8 operation panel, 10 medical care apparatus, 13 control unit, 41 wireless communication unit, 43 wired communication unit, 45 attachment unit, 100 communication device, 111 control unit, 112 storage unit, 113 camera, 114 microphone, 115 touch display, 116 wireless communication unit, 117 wired communication unit, 1000 medical care system.

## Claims

1. A medical care system comprising:
a medical care apparatus having a plurality of functions; and
a communication device capable of communicating with the medical care apparatus,
the medical care apparatus including a first operation unit through which each of the plurality of functions can be set,
the communication device including a second operation unit through which at least one of the plurality of functions can be set in a more detailed manner than through the first operation unit, and
the plurality of functions including specific functions, and settings by the second operation unit for the specific functions being restricted.

2. The medical care system according to claim 1, wherein
the medical care apparatus includes a movable unit configured to change at least one of a position and a posture of the medical care apparatus, and
the specific functions are for driving the movable unit.

3. The medical care system according to claim 1 or 2, wherein
when the medical care apparatus and the communication device are not communicating with each other,
in the first operation unit, a setting for each of the plurality of functions is allowed, and
in the second operation unit, a setting for each of the plurality of functions is restricted, and
when the medical care apparatus and the communication device are communicating with each other,
in the first operation unit, settings for the specific functions among the plurality of functions are allowed and settings for other functions different from the specific functions among the plurality of functions are restricted, and
in the second operation unit, the settings for the other functions are allowed and the settings for the specific functions are restricted.

4. The medical care system according to any one of claims 1 to 3, wherein
the medical care apparatus includes a first visibility changing unit configured to change visibility of the first operation unit,
the communication device includes a second visibility changing unit configured to change visibility of the second operation unit, and
when the medical care apparatus and the communication device are communicating with each other,
the first visibility changing unit is configured to lower the visibility of the first operation unit associated with other functions different from the specific functions among the plurality of functions below the visibility of the first operation unit associated with the specific functions, and
the second visibility changing unit is configured to lower the visibility of the second operation unit associated with the specific functions below the visibility of the second operation unit associated with the other functions.

5. The medical care system according to any one of claims 1 to 4, wherein
the communication device is a mobile terminal capable of wirelessly communicating with the medical care apparatus, and
the communication device is configured to communicate with a medical care apparatus among a plurality of medical care apparatuses that is closest to the communication device.

6. The medical care system according to any one of claims 1 to 5, wherein
the communication device includes a storage unit configured to store setting data about other functions different from the specific functions among the plurality of functions, and
when the medical care apparatus and the communication device are communicating with each other, the other functions are set in the medical care apparatus based on the setting data stored in the storage unit.

7. The medical care system according to claim 6, wherein
the communication device includes a voice authentication unit configured to authenticate a user by voice authentication,
the storage unit is configured to store the setting data and information, which allows identification of the user, such that the setting data and the information are associated with each other, and
when the medical care apparatus and the communication device are communicating with each other, the other functions are set in the medical care apparatus based on the setting data associated with a user authenticated by the voice authentication unit.

8. The medical care system according to claim 6 or 7, wherein
the communication device includes a facial configuration authentication unit configured to authenticate a user by facial configuration authentication,
the storage unit is configured to store the setting data and information, which allows identification of the user, such that the setting data and the information are associated with each other, and
when the medical care apparatus and the communication device are communicating with each other, the other functions are set in the medical care apparatus based on the setting data associated with a user authenticated by the facial configuration authentication unit.

9. The medical care system according to any one of claims 1 to 8, wherein
the medical care apparatus includes an attachment unit to which the communication device can be attached, and
when the communication device is attached to the attachment unit, the medical care apparatus and the communication device are brought into communication with each other.

10. The medical care system according to any one of claims 1 to 9, wherein the communication device is configured to update at least one of a program and data of the medical care apparatus when the medical care apparatus and the communication device are communicating with each other.

11. The medical care apparatus according to any one of claims 1 to 10.

12. The communication device according to any one of claims 1 to 10.

13. A method of preventing an erroneous operation of a medical care apparatus having a plurality of functions, the method comprising:
performing an operation by which a setting for each of the plurality of functions can be made;
communicating with a communication device by which a setting for at least one of the plurality of functions can be made in a more detailed manner than in the performing an operation; and
restricting settings by the communication device for specific functions among the plurality of functions.
